# EUROPEAN PATENT APPLICATION

(11) **EP 0 597 810 A2**
(43) Date of publication of application: **18.05.1994**
(21) Application number: 93830448.2
(22) Date of filing: 05.11.1993
(51) Int. Cl.: A61F 2/04, A01N 1/02, C12N 5/00, A61L 27/00

(54) **Epithelium-tubular carrier biostructure and method for the preparation thereof**

(30) Priority: 09.11.1992 IT RM920813
(71) Applicant: ISTITUTO NAZIONALE PER LA RICERCA SUL CANCRO, I-16132 Genova (IT)
(72) Inventor: Cancedda, Ranieri, Istituto Nazionale Per, I-16132 Genova (IT); De Luca, Michele, Istituto Nazionale Per, I-16132 Genova (IT)
(74) Representative: de Simone, Domenico

(57) **Abstract**

A biostructure is described, comprising:
- a substantially tubular shaped carrying mean made out of an inert, flexible and non toxic material, not effective as substrate for cells and tissues to adhere and grow; and
- an *in vitro* cultured epithelium, having at least the same length of said carrying mean, or a larger lenght, being wound as a spiral on said carrying mean, so that the basal layer of said epithelium is turned to the exterior and the apical layer of said epithelium is turned to said carrying mean.

The biostructure may be advantageously used in the surgery of urethral deformities.

## Description

The invention concerns a biostructure, comprising a tubular carrier, covered by an *in vitro* cultured epithelium or lining mucosa, to be used in the surgery of urethral deformities.

Among urethral deformities, hypospadias is a congenital pathology affecting 0.3% of male newborn humans. Affected subjects show either a missing or a misformed urethra, ending at the ventral surface of penis. Approximately 20% of affected subjects shows a severe deformity form, known as posterior hypospadias, wherein the urethral meatus is located at the bese of penis.

Urethral transplantation methods are known, using autologous grafts from bladder mucosa, as described by Coleman, J.W., J. Urol., 125, 708 (1981), and Li, Z.C., Zheng, Y.H, Sheh, Y.X and Capo, J.F., Urol. Clin. N. Amer., 8, 457 (1981). These methods require to submit the patient to a surgery operation, under total anesthesia conditions, and to long times of hospitalization, due to graft procedures.

Duckett, J.W. described a method, in "Adult and Pediatric Urology, Y. Gillewater and al. Ed., Year Book Medical, volume 1, pages 1880-1915 (1987), using an autologous graft from prepuce skin epithelia. Also there, a surgery operation under total anesthesia is involved; moreover, the obtained epithelium is often too small for transplantation purposes. Epidermal grafts were also used, having heavy secondary effects due to the presence of piliferous folliculi as well as of sebaceous cells, as described by Devim, C. Jr, and Horton C.E., J. Urol., 118, 188 (1977).

The inventors described recently an urethra transplantation method, wherein an *in vitro* cultured epithelium from autologous patient cells was used (Romagnoli, G., De Luca, M., Faranda, F., Bandelloni,R, Franzi, A.T., Cataliotti, F and Cancedda R., New Engl. Med. 323, 527 (1990)). Such method, altough showing the practicability to use *in vitro* cultured epithelia for urethra transplantation operations, cannot usually be applied. As a matter of fact, the method comprises to culture the epithelium *in vitro* up to the size required to reconstruct the urethral tube, depending upon patient's age and penis' size and, thereafter, to graft said epithelium onto the internal surface of the penis, previously "opened" by means of a longitudinal incision along its ventral section. At least ten days have to elapse, waiting for the transplanted epithelium adhering and "taking". When positive symptoms are detected, the transplanted epithelium is cut away together with the underlying connective tissue, sutured up to have a hollow duct and then the two skin flaps are sutured onto the urethra. Obviously this operation cannot be carried out on a large scale basis, due to long times involved, to patient's pains during the hospitalisation, to the need of two close total anesthesia procedures and to high organic contamination risks of grafted tissue during adhering and "taking" onto the connective tissue, before of penis suturing.

The inventors have found that a biostructure, consisting of an inert and biocompatible tubular carrier, on which an *in vitro* cultured epithelium flap is wound, obtained by an autologous biopsy, can effectively and advantageously be used for urethra deformity surgery, by means of only one short operation, thus avoiding close total anesthesia precedures, a long patient's hospitalisation after the operation and contaminations risks.

It is therefore an object of this invention a biostructure, comprising:
- a substantially tubular shaped carrying mean made out of an inert, flexible and non toxic material, not effective as substrate for cells and tissues to adhere and grow; and
- an *in vitro* cultured epithelium, having at least the same length of said carrying mean, or a larger lenght, being wound as a spiral on said carrying mean, so that the basal layer of said epithelium is turned to the exterior and the apical layer of said epithelium is turned to said carrying mean.

"Inert material" means a material, that will cause neither allergy nor rejection reactions, when grafted to an host organism; "basal layer" means the surface of an epithelium or of a lining mucosa, that is physiologically turned toward the connective tissue; "apical layer" means the surface of an epithelium or of a lining mucosa, that is physiologically turned to the exterior.

Preferably, said carrying mean has an internal diameter in the range 6-25 frenches, more preferably in the range 10-18 frenches; and a length in the range 2-15 cm; and such inert material has a tickness of 1 mm, or less.

According to a preferred embodiment of the invention said carrier comprises a Goretex® tube.

According to another preferred embodiment of the invention, said *in vitro* cultured epithelium is obtained from an autologous biopsy of an epithelium tissue, preferably an urethral mucosa.

Further according to the invention, said epithelium is wound up on said carrying mean as a spiral for at least one complete turn, preferably for 2-4 complete turns.

It is a further object of this invention a method to obtain a biostructure, comprising the steps as follows:
- to cultivate said epithelium *in vitro* until a flap is obtained, said flap having at least the same length of said carrying mean, or a larger lenght;
- to detach said epithelium flap from its culture substrate, by using detaching means that are unable to dissociate cell-cell interactions; preferably said detaching means being neutral protease dispase;
- to wound up said epithelium flap on said carrying mean in order to have the basal layer turned to the exterior and the apical layer turned to said carrying mean.

According to another embodiment of the invention the method comprises:
- to cultivate said epithelium *in vitro* until a flap is obtained, said flap having at least the same length of said carrying mean, or a larger lenght;
- to detach said epithelium flap from its culture substrate, by using detaching means that are unable to dissociate cell-cell interactions; preferably said detaching means being neutral protease dispase;
- to freeze said epithelium flap to a temperature of at least -80°C, in the presence of a cryopreserving agent; preferably at least -110°C;
- to thaw said epithelium flap by incubating at 37°C; and
- to wound up said epithelium flap on said carrying mean in order to have the basal layer turned to the exterior and the apical layer turned to said carrying mean.

According to a preferred embodiment, said epithelium is fastened to each end of the said carrying mean by means of metal stitches.

The invention will be described according to the following not limiting example.

### EXAMPLE 1

2 mm² biopsies of urethral mucosa are excised under local anesthesia from the urethral meatus from 8 patients, from 2 up to 14 year old, who suffered severe posterior hypospadias. One patient's biopsy is excised from a flap of the prepuce epithelium. Biopsies are transferred to a laboratory under sterility conditions, having being dipped into Dulbecco's medium, containing 10% of FCS and 4 mM of glutamine. Biopsies are used within 21-48 hrs to grow and obtain urethral mucosa flaps *in vitro*. Cultivation methods are described in the references as follows:
- De Luca, M., Albanese, E., Megna, M., Cancedda, R.,Mangiante, P.E., Cadoni, A. and Franzi, A.T.: "Evidence that Human Oral Epithelium Reconstituted *in vitro* and transplanted onto Patients with Oral Mucosa Defects Retains Properties of the Original Donor Site; Transplantation, 50:454 (1990);
- Romagnoli, G., De Luca, M., Faranda, F., Bandelloni, R., Franzi, A.T., Cataliottti, F. and Cancedda, R.: "Treatment of Posterior Hypospadias by the Autologous Graft of Cultured Urethral Epithelium", N. Engl. J. Med., 323:527 (1990); and
- Green, H., Khinde, O. and Thomas, J., Proc. Natl. Acad. Sci. USA, 76, 5665 (1979).

After 15-18 days grafts are obtained from secondary confluent cultures, by detaching treatment with neutral protease Dispase II, as described in the references as above.

Flaps are used immediately, or, alternatively, frozen up to -110°C, according to the method described in EP N. 2.964.475.

Layers of urethral mucosa, either directly obtained from the Dispase II treatment, or thawed, are wound up as a spiral on a Goretex® tube (supplied by Gore & Assoc., Inc., US), by rolling it gently with the help of forceps, in order to turn the flap basal layer outside. In some cases, the mucosa is fastened to each end of the Goretex® tube by means of metal stitches. The obtained biostructures are maintained for at least 24 hours at a temperature ranging from 4 to 20°C, under sterile conditions.

The obtained biostructure is grafted to the same patient to whom the biopsy was carried out. A Foley catether is introduced in the urethra meatus and a ligth incision is carried out in order to provide a sub-cutaneous duct from the meatus to the glans navicular fossa, along the penis longitudinal axis, to prevent blood losses, that would negatively affect the "taking" of *in vitro* cultured mucosa. The sub-cutaneous duct is widened by means of forceps in order to minimize the friction of the epithelium basal layer when the biostructure is to be grafted into the duct.

When the biostructure is finally inserted into the duct, a further slip or displacement of the biostructure is to be avoided in order not to damage the epithelium cell basal layer. The biostructure epithelium is sutured to the urethra meatus from one side, and to the navicular fossa, previously surgery treated, from the other side. Then the catether is inserted in the new urethra into the duct of the Goretex® tube. The total surgery operation lasts 30 minutes appr.

After ten days the Foley catheter is removed and a spontaneous urine discharge occurs through the Goretex® tube. After further ten days the Goretex® tube is removed through a gentle extraction procedure.

In all of treated subjects, the new urethra shows to be correctly coated with a mucosa. All patients show correct urine discharge and penis erection functions. Uretroscopies performed 24 months after the operation show a normal evolution of the transplanted tissue.

## Claims

1. A biostructure, comprising:
- a substantially tubular shaped carrying mean made out of an inert, flexible and non toxic material, not effective as substrate for cells and tissues to adhere and grow; and
- an *in vitro* cultured epithelium, having at least the same length of said carrying mean, or a larger lenght, being wound as a spiral on said carrying mean, so that the basal layer of said epithelium is turned to the exterior and the apical layer of said epithelium is turned to said carrying mean.

2. A biostructure according to claim 1, wherein said carrying mean has an internal diameter ranging from 6 to 25 frenches, more preferably from 10 to 18 frenches, and a length ranging from 2 to 15 cm; and said inert material is 1 mm thick, or less.

3. A biostructure according to any of previous claims, wherein said carrying mean comprises a tube made of Goretex®.

4. A biostructure according to any of previous claims, wherein said *in vitro* cultured epithelium is derived from an autologous biopsy of an epithelium tissue, preferably an urethral mucosa.

5. A biostructure according to any of previous claims, wherein said epithelium is wound up as a spiral on said carrying mean for at least one complete turn; and more preferably for 2-4 complete turns.

6. A method for the preparation of a biostructure according to any of previous claims, comprising the steps as follows:
- to cultivate said epithelium *in vitro* until a flap is obtained, said flap having at least the same length of said carrying mean, or a larger lenght;
- to detach said epithelium flap from its culture substrate, by using detaching means that are unable to dissociate cell-cell interactions; preferably said detaching means being neutral protease dispase;
- to wound up said epithelium flap on said carrying mean in order to have the basal layer turned to the exterior and the apical layer turned to said carrying mean.

7. A method for the preparation of a biostructure according to any of previous claims, comprising the steps as follows:
- to cultivate said epithelium *in vitro* until a flap is obtained, said flap having at least the same length of said carrying mean, or a larger lenght;
- to detach said epithelium flap from its culture substrate, by using detaching means that are unable to dissociate cell-cell interactions; preferably said detaching means being neutral protease dispase;
- to freeze said epithelium flap to a temperature of at least -80°C, in the presence of a cryopreserving agent; preferably at least -110°C;
- to thaw said epithelium flap by incubating at 37°C; and
- to wound up said epithelium flap on said carrying mean in order to have the basal layer turned to the exterior and the apical layer turned to said carrying mean.

8. A method according to claims 6 or 7, wherein said epithelium flap is fastened to each end of said carrier by means of metal stitches.
